# EUROPEAN PATENT APPLICATION

(11) **EP 0 547 008 A1**
(43) Date of publication of application: **16.06.1993**
(21) Application number: 92830639.8
(22) Date of filing: 25.11.1992
(51) Int. Cl.: C07H 19/04, C07H 21/00, C12N 15/11

(54) **New antisense oligonucleotides**

(30) Priority: 26.11.1991 IT RM910890
(71) Applicant: ISTITUTO DI RICERCHE DI BIOLOGIA MOLECOLARE P. ANGELETTI S.P.A., 00040 Pomezia (IT)
(72) Inventor: Iribarren, Adolfo, I-00124 Roma (Casalpalocco) RM (IT)
(74) Representative: Di Cerbo, Mario

(57) **Abstract**

Antisense oligonucleotides, characterized by the fact that they are 2′-deoxy-2′-C-hydrocarbyl oligonucleotides with configuration 2′R or 2′S and that they have the following general formula (1): 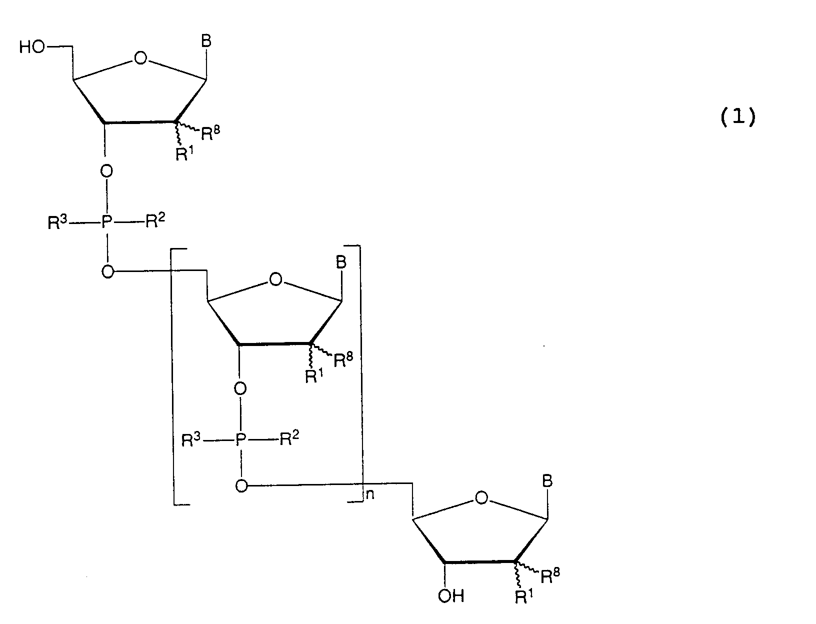
wherein
n is an integer comprised between 0 and 200;
R1 represents hydrocarbyl residues, such as alkyl, alkenyl and aralkyl, which are substituted at least once by groups possessing functions;
B represents a mono or polynuclear heterocyclic base;
R2 and R3 represent, independently from each other, heteroatom optionally bonded to hydrogen, hydrogen, or residues R1, optionally bonded to phosphor by heteroatom; and
R8 represents heteroatom optionally bonded to hydrogen, hydrogen or residues R1 optionally bonded to carbon by heteroatom.

## Description

The present invention has as its subject new chemically modified antisense oligonucleotides, and more precisely 2'-deoxy-2'-C-hydrocarbyloligonucleotides, both with 2'R and with 2'S configuration.

As is known, the specificity of the pairing of complementary bases between antisense oligonucleotides and the target sequence makes antisense oligonucleotides suitable for applications ranging from basic scientific research in molecular biology, to therapeutics and diagnostics.

In these applications, however, there is the problem of the stability of oligonucleotides in vivo. In fact, oligonucleotides which are not chemically modified are subject to enzymatic hydrolysis by nucleases, with extremely rapid reaction kinetics. This phenomenon, given that the strategy used in application of these molecules is based on their nucleotidic sequence, greatly limits or even compromises their use for the purposes desired.

Naturally, the chemical modifications introduced, as well as reducing the rate of enzymatic hydrolysis in vivo, must be such as not to prejudice other important properties which the oligonucleotides are required to show in order to perform their function in the applications mentioned. In particular, the following properties must not be impaired:
- the capacity for hybridization with the target nucleic acid (in particular, chemical modifications must not impair the hydrogen bonding capability of the bases. Furthermore, in order not to disrupt the formation of Watson-Crick base pairing, it is preferable that any chemical modification that is made should be rather conservative);
- the capacity to cross the cell membrane and reach the target sequence within the cell;
- the capacity to inhibit expression of the gene coded for by the hybridized mRNA; and
- the capacity to perform selective binding (nonspecific binding has to be minimized in order to avoid a decrease in the concentration and thus in the potency of the antisense oligonucleotide).

The state of the art records a number of attempts to chemically modify the antisense fragments for the purposes mentioned above. In this regard, those modifications providing for substitution on the 2'-carbon of the sugar ring have proved to be particularly interesting. Remarkable results have been obtained by use of 2'-O-alkyl oligonucleotides. These molecules have shown to have increased resistance to nucleases, to form very stable hybrids and to minimize nonspecific binding.

However, the use of 2'-O-alkyl-oligonucleotides is not entirely satisfactory. The main drawback to be found in the use of these chemically modified oligonucleotides is the complexity of the reactions required to prepare the building blocks necessary for their synthesis. A direct consequence of this complexity is the complication of the installation aspects connected to the realization of the related manufacturing process at an industrial level. A further drawback which has a negative effect on the use of 2'-O-alkyl-oligonucleotides is the high cost of certain starting products necessary for their synthesis.

New chemically modified oligonucleotides have now been found which permit all the above mentioned drawbacks to be overcome, furthermore giving additional advantages which will be made clear in the following.

The oligonucleotides according to the present invention are characterized by the fact that they are 2'-deoxy-2'-C-hydrocarbyl-oligonucleotides with configuration 2'R or 2'S and that they have the following general formula (1): 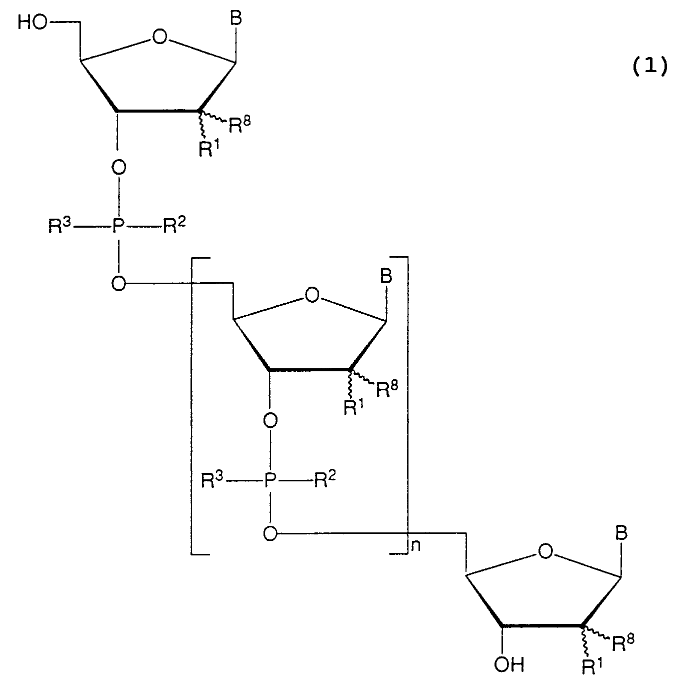
wherein
n is an integer comprised between 0 and 200;
R1 represents hydrocarbyl residues, such as alkyl, alkenyl and aralkyl, which are substituted at least once by groups possessing functions;
B represents a mono or polynuclear heterocyclic base;
R2 and R3 represent, independently from each other, heteroatom optionally bonded to hydrogen, hydrogen, or residues R1, optionally bonded to phosphor by heteroatom; and
R8 represents heteroatom optionally bonded to hydrogen, hydrogen or residues R1 optionally bonded to carbon by heteratom.

The residues R1 are preferably chosen from the group comprising methyl, ethyl, propyl, allyl and benzyl. These residues are preferably substituted with functional groups chosen from among -NH₂, -OH, and -CO.

The residue B preferably represents purine derivatives such as adenine, hypoxanthine and guanine, or pyrimidine derivatives, such as cytosine, uracil and thymine.

The heteroatoms represented by R2 and R3 are preferably chosen from the group comprising oxygen and sulphur.

Among the heteroatoms which bond the residues of type R1 to the phosphorus, the oxygen atom is preferred to represent the residues R2 and R3.

The present invention is not limited to 2'-deoxy-2'-C-hydrocarbyl-oligonucleotides per se, but also extends to antisense 2'-deoxy-2'-C-hydroxycarbyloligonucleotides for therapeutic and diagnostic use.

A further subject of the present invention is the compound representing the building block to be used as a starting product in the synthesis of chemically modified oligonucleotides according to the present invention.

These are compounds characterized by the fact that they have the following structural formula (2): 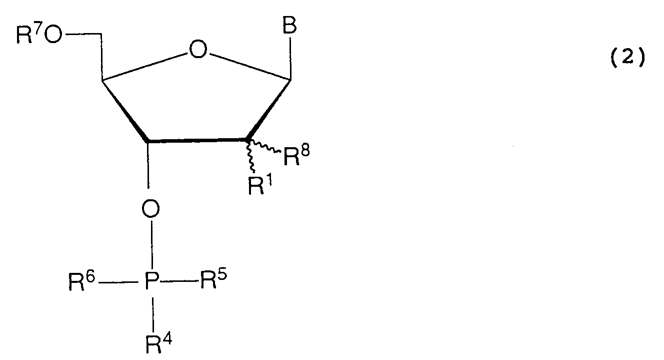
wherein
R1 represents hydrocarbyl residues, such as alkyl, alkenyl and aralkyl, which are optionally substituted at least once by groups possessing functions;
B represents a mono or polynuclear heterocyclic nitrogenous base;
R4, R5 and R6 represent, independently from each other, heteroatom optionally bonded to hydrogen, hydrogen, or residues R1, optionally bonded to phosphor by heteroatom, one of these residues being optionally absent;
R7 represents hydrogen or a protective group;
R8 represents heteroatom optionally bonded to hydrogen, hydrogen or residues R1 optionally bonded to carbon by heteratom,
and by the fact that they have a 2'R or 2'S configuration.

The residues R1 are preferably chosen from the group comprising methyl, ethyl, propyl, allyl and benzyl. These residues are preferably substituted with functional groups chosen from among -NH₂, -OH, and -CO.

B preferably represents purine derivatives such as adenine, hypoxanthine and guanine, or pyrimidine derivatives, such as cytosine, uracil and thymine.

The heteroatoms represented by R4, R5 and R6 are preferably chosen from the group comprising oxygen and sulphur.

Among the heteroatoms which bond the residues of type R1 to the phosphorus, the oxygen atom is preferred to represent the residues R4, R5 and R6.

The groups R1 and B of the building block can be protected, for example with benzoyl. The protective group represented by R7 can advantageously be 4,4'-dimethoxytriphenylmethyl.

The invention also refers to the process for the synthesis of said 2'-deoxy-2'-C-hydrocarbyloligonucleotides starting from said building blocks.

Up to this point, a description of a general nature has been given of the subjects of the present invention. With the aid of the following examples, which refer to the synthesis of specific embodiments of the invention, further details will now be given to clarify the aims, characteristics and advantages thereof. In the examples the annotations (2'R) and (2'S) indicate the configurations 2'R and 2'S respectively (the configuration 2'S is more advantageous as regards hybridization).

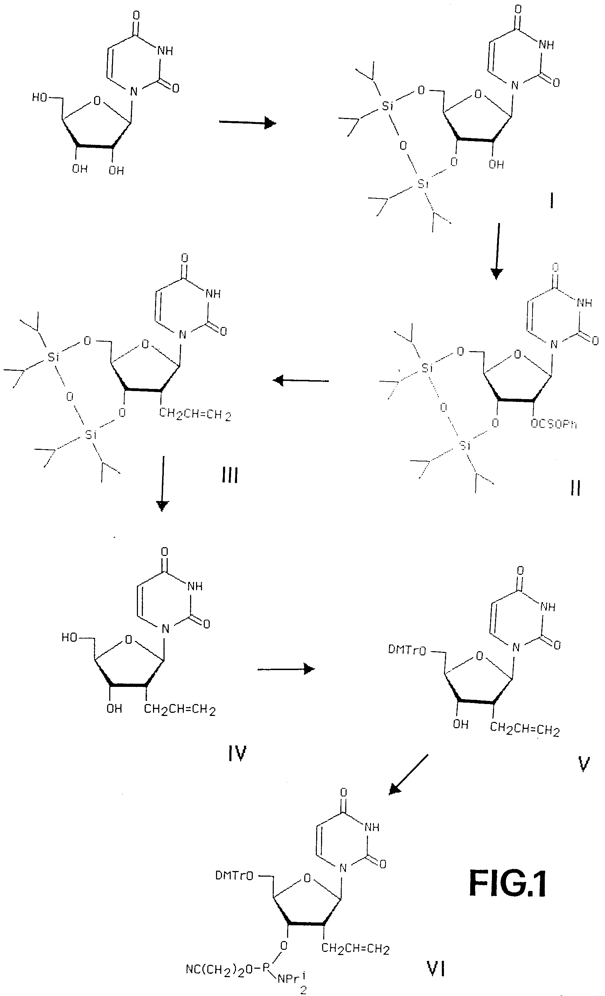

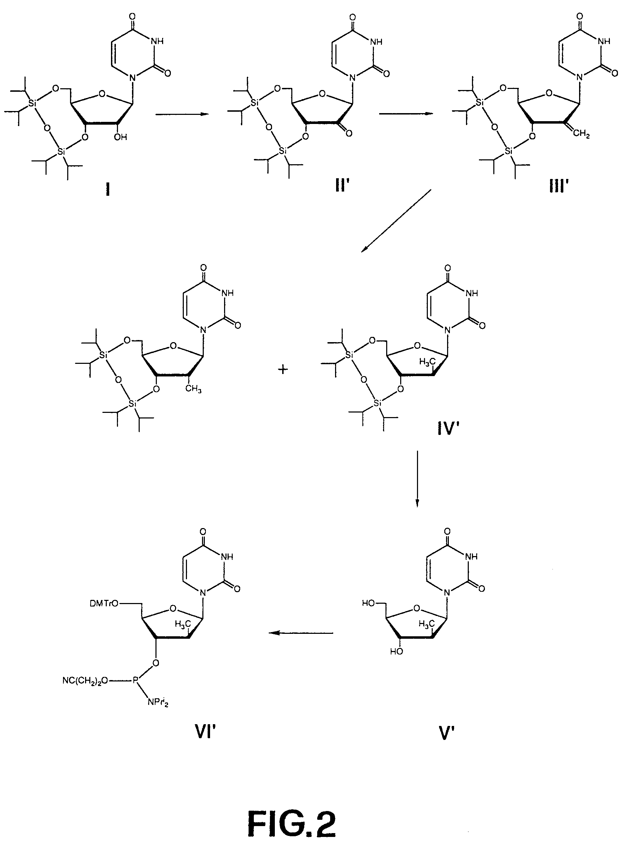

Figures 1 and 2 represent the reaction schemes for the synthesis of the building blocks of example 1 and 3 respectively.

### EXAMPLE 1

### SYNTHESIS OF (2'R)-2'-DEOXY-2'C-ALLYLURIDINE BUILDING BLOCK

Synthesis is illustrated with reference to the intermediates obtained at each stage of the reaction scheme indicated in the sole figure enclosed.

### 3',5'-O-(Tetraisopropyldisiloxane-1,3-diyl)-uridine (I)

Uridine (14g, 57 mmol) was dissolved in dry pyridine (100 ml) and a solution of 1,3-dichloro-1,1,3,3-tetraisopropyldisiloxane (20 ml) in 1,2-dichloroethane (20 ml) was added dropwise at 0°°C. The reaction mixture was stirred at room temperature for 3 hours. Methanol (15 ml) was added and the mixture stirred until a clear solution was obtained. The solution was concentrated **in vacuo**, and the residue was extracted with ethylacetate (800 ml) and 1 M aqueous sodium bicarbonate (500 ml). The organic layer was dried on sodium sulfate, filtered and evaporated to afford 32.77 g of product as a foam.

### 3',5'-O-(Tetraisopropyldisiloxane-1,3-diyl)-2'-O-phenylthionoformate uridine (II)

To a stirred solution of (I) (3 g) and dimethylaminopyridine (120 mg) in dry pyridine (60 ml) was added O-phenylchlorothionoformate (1.2 ml). The reaction mixture was stirred overnight at room temperature. The solvent was evaporated **in vacuo** and the residue extracted in dichloromethane (200 ml) and water (200 ml). The organic phase was dried on sodium sulfate, filtered and evaporated. The resulting syrup was purified by column chromatography on silica gen eluting with petrol/ethyol acetate (4:1 v/v) affording the pure compound (2.34 g, 71% yield).

### 3',5'-O-(Tetraisopropyldisiloxane-1,3-diyl)-(2'R)-2'-deoxy-2'-C-allyluridine (III)

A solution of compound (II) (1.31 g) in dry toluene (2.1 ml) was degassed and treated with AIBN (52 mg) and allyltributyltin (1.3 ml). The reaction mixture was stirred at 80°C for 5 hours. The solvents were removed **in vacuo** and the residue was purified by column chromatography on silica gel using petrol/ethyl acetate (5:1 v/v) as eluant. Compound (III) (0.53 g, 49% yield) was obtained as a solid foam.

### (2'-R)-2'-Deoxy-2'-C-allyluridine (IV)

A solution of tetrabutylammomium fluoride in THF (1M, 4 ml) was added to a solution of compound (III) in THF (30 ml). The solution was stirred at room temperature and after 5 minutes the reaction quenched with pyridine/methanol/water (3:3:1 v/v/v, 0.6 ml). The suspension obtained by pouring this solution into piridinium form Dowex 50Wx4-200 resin was stirred for 10 minutes, the resin filtered off and washed with methanol. The filtrate and washings were pooled together and evaporated to dryness **in vacuo** to afford a syrup. Purification of this residue by column chromatography on silica gel eluting with dichloromethane/methanol (9:1 v/v) yielded the pure compound (0.49 g, 98% yield).

### 5'-O-Dimethoxytrityl-(2'R)-2'-deoxy-2'-C-allyluridine (V)

Compound (IV) (0.48 g) was dissolved in dry pyridine (17 ml) and treated with dimethoxytrityl chloride (0.74 g), triethylamine (0.35 ml) and 4-dimethylaminopyridine (10 mg). The resulting solution was stirred overnight at room temperature. Silica gel thin layer chromatography showed complete reaction. Water (50 ml) was added and the mixture extracted with dichloromethane (2x100 ml). The combined organic phases were dried on sodium sulfate, filtered and evaporated **in vacuo**. The resulting syrup was purified using column chromatography on silica gel with dichloromethane/methanol (99:1 v/v) as eluant affording the pure compound (0.77 g, 75% yield).

### 5'-O-Dimethoxytrityl-(2'R)-2'-deoxy-2'-C-allyluridine-3'-O-(2-cyanoethyl-N,N-diisopropylphosphoramidite) (VI)

To a solution of compound (V) (0.77g) and N,N-diisopropylethylamine (2.2 ml) in 1,2-dichloroethane (20 ml) under argon was added dropwise 2-cyanoethoxy-N,N-diisopropylaminochlorophosphine (0.5 ml). The reaction was stirred overnight at room temperature and then quenched by addition of methanol (0.2 ml). After 5 minutes, dichloromethane (100 ml) was added and the solution washed with saturated sodium chloride (2x100 ml). The organic layer was dried on sodium sulfate, filtered and evaporated to dryness **in vacuo**. The residue was purified by column chromatography on silica gel eluting with a gradient of methanol from 0% to 2% in dichloromethane containing 1% of triethylamine. The pure compound (0.57 g, 55% yield) was obtained as a solid white foam.

The above synthesis is notably simpler and less costly than conventional ones. For example, the synthesis of the building block 2'-O-allyl-guanosine would have required as many as 12 reaction stages starting from 6-chloroguanosine, which, as it is known, is an extremely expensive product.

### EXAMPLE 2

### SYNTHESIS OF 2-C-ALLYLOLIGODEOXYNUCLEOTIDES

A twentymer homopolymer of 2'-C-allyluridine was synthesised using standard conditions on an Applied Biosystem synthesizer. The concentration of the building blocks and of the activator, 5-(4-nitrophenyl)-1H-tetrazole were 0.1 M and 0.15 M, respectively. The condensation wait time was increased to 7 minutes. The overall coupling yield was 78% (99% per coupling).

### EXAMPLE 3

### SYNTHESIS OF 5'-O-DIMETHOXYTRITYL-(2'S)-2'-DEOXY-2'-C-METHYL URIDINE-3'-O-(2-CYANOETHYL-N,N-DIISOPROPYLPHOSPHORAMIDITE) [(2'S)-2'-DEOXY-2'-C-METHYLURIDINE BUILDING BLOCK]

Synthesis is illustrated with reference to the intermediates obtained at each stage of the reaction scheme indicated in figure 2.

### 3',5'-O-(Tetraisopropyldisiloxane-1,3-diyl)-2'-keto-uridine (II')

To an ice cold stirred suspension of the preformed complex of CrO₃/pyridine/acetic anhydride (molar ratio 1:2:1) (63 g), in 100 ml of 1,2-dichloromethane was added compound I (29 g).

The reaction mixture was stirred at 0°C for 15 min and the dark brown solution was applied on a silica gel column for a percolation with ethyl acetate as eluant. After removal of the solvent in vacuo the desired product was obtained as a white solid (22 g, 75%).

### 3',5'-O-(Tetraisopropyldisiloxane-1,3-diyl)-2'-deoxy-2'-methylidene-uridine (III')

To an ice cold suspension of methyltriphenylphosphonium bromide (11 g) in 60 ml of dry THF, was added under anhydrous conditions a solution of 1-butylithium (1.6 M in hexane; 31.2 ml). After stirring for 1 h at 0°C, 3', 5'-O-(tetraisopropyldisiloxane-1,3-diyl)-2'-keto-uridine (10 g) in 30 ml of dry THF was added dropwise over 15 min. The resulting reaction mixture was stirred at room temperature for 3 h. After completion of the reaction, 20 ml of 1 M aqueous NH₄Cl was added and the reaction mixture was stirred for 10 more min. The mixture was diluted with 300 ml of ethyl acetate and washed with water. The organic layer was dried over Na₂SO₄, filtered and concetrated **in vacuo**. The resultant dark red syrup was purified by silica gel column chromatography using petrol/ethyl acetate (3 : 1 v/v) as eluant. Pure title compound was obtained as a white solid (11.10 g, 55.5%).

### (2'S)-2'-deoxy-2'-C-methyluridine) (IV')

3',5'-O-(Tetraisopropyldisiloxane-1,3-diyl)-2'-deoxy-2'-methylidenuridine (1.61 g) and 5% Pd on CaCO₃ (0.5 g) in ethyl acetate (100 ml) were stirred overnight at room temperature under hydrogen atmosphere (6 bar). The reaction mixture was filtered through celite and the filtrate evaporated to dryness to afford a mixture of title compounds in a 3/1 ratio (1.6 g).

### (2'S)-2'-Deoxy-2'-C-methyluridine (V')

A mixture of 3',5'-O-(Tetraisopropyldisiloxane-1,3-diyl)-(2'R) and (2'S)-2'-deoxy-2'-C-methyluridine (1.4 g; 1/3) were treated as indicated for compound IV.

The crude product was purified by column chromatography on silica gel eluting with methylene chloride:methanol 10:1. A purified mixture of (2'R) and (2'S)-2'-deoxy-2'-C-methyluridine was obtained in 85% yield (0.72 g). This mixture was resolved by column chromatography (eluted with methylene chloride:methanol 15:1) in order to get pure title compound.

### 5'-O-Dimethoxytrityl-(2'S)-2'-deoxy-2'-C-methyluridine-3'-O-(2-cyanoethyl-N,N-diisopropylphosphoramidite) (VI')

(2'S)-2'-Deoxy-2'-C-methyluridine was dimethoxytritylated and phosphitylated as previously described for compounds V and VI, respectively.

### EXAMPLE 4

### HYBRIDIZATION AND STABILITY TO NUCLEASES OF A MODIFIED OLIGONUCLEOTIDE ACCORDING TO THE INVENTION

The basic requirement that new antisense fragments have tu fulfil is to possess good hybridization characteristics. Therefore, the affinity of the binding between these fragments and the complementary DNA sequence has been tested.

The following oligonucleotide:
dC(CCUCUCCUUCUCCCUCCU)_{2'S'}
carrying (2'S)-2'-deoxy-2'-C-methyl nucleotides in all the positions but the 3'-end, has been synthesized using standard synthesis protocols on an Applied Biosystems synthesizer model 380 B, through β-cyanoethyl phosphoramidite chemistry. The coupling time was incremented to 7 min.

The melting point of an equimolecular mixture of this modified oligonucleotide (3/uM) and its complementary DNA sequence in 0.1 M sodium chloride solution was measured at 260 nm. The same experiment was done using the same non-modified DNA sequence. The results obtained indicated that the modified oligonucleotide forms a more stable hybrid than the natural DNA fragment since the melting points are 38.6°C and 35.5°C, respectively.

The second important property of an antisense fragment is its stability to nucleases. With regard to this, three different nucleases were tested: P1, Bal 31 and Mung Bean. the sequence and modification were the same as described above. The (2'S)-2'-deoxy-2'-C-methyl oligonucleotide showed to be resistant to these nucleases, since the same concentration that produced complete degradation of the non-modified sequence did not affect this modified one.

## Claims

1. Oligonucleotides, characterized by the fact that they are 2'-deoxy-2'-C-hydrocarbyl oligonucleotides with configuration 2'R or 2'S and that they have the following general formula (1): 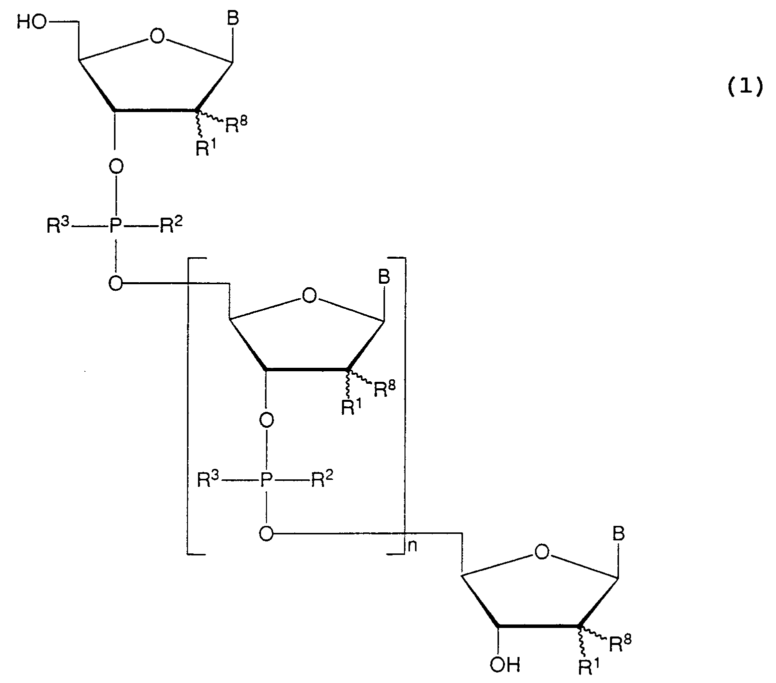 wherein
n is an integer comprised between 0 and 200;
R1 represents hydrocarbyl residues, such as alkyl, alkenyl and aralkyl, which are substituted at least once by groups possessing functions;
B represents a mono or polynuclear heterocyclic base;
R2 and R3 represent, independently from each other, heteroatom optionally bonded to hydrogen, hydrogen, or residues R1, optionally bonded to phosphor by heteroatom; and
R8 represents heteroatom optionally bonded to hydrogen, hydrogen or residues R1 optionally bonded to carbon by heteratom.

2. Oligonucleotides according to claim 1, in which the residue R1 is preferably chosen from the group comprising methyl, ethyl, propyl, allyl and benzyl.

3. Oligonucleotides according to claims 1 or 2, in which the R1 hydrocarbyl groups are substituted with functional groups chosen from among -NH₂, -OH, and -CO.

4. Oligonucleotides according to any one of the preceding claims, in which B represents a purine derivative.

5. Oligonucleotides according to claim 4, in which the purine derivative is chosen from the group comprising adenine, hypoxanthine and guanine.

6. Oligonucleotides according to any one of claims 1 to 3, in which B represents pyrimidine derivatives.

7. Oligonucleotides according to claim 6, in which the pyrimidine derivative is chosen from the group comprising cytosine, uracil and thymine.

8. Oligonucleotides according to any one of the preceding claims, in which the heteroatoms represented by R2 and R3 are preferably chosen from the group comprising oxygen and sulphur.

9. Oligonucleotides according to any one of the preceding claims, in which among the heteroatoms which bond the residues of type R1 to the phosphorus, the oxygen atom is preferred to represent the residues R2 and R3.

10. Antisense oligonucleotides, for therapeutic and diagnostic use, characterized in that they are 2'-deoxy-2'-C-hydrocarbyl oligonucleotides according to claims 1 to 9.

11. Compounds, characterized in that they have the following structural formula (2): 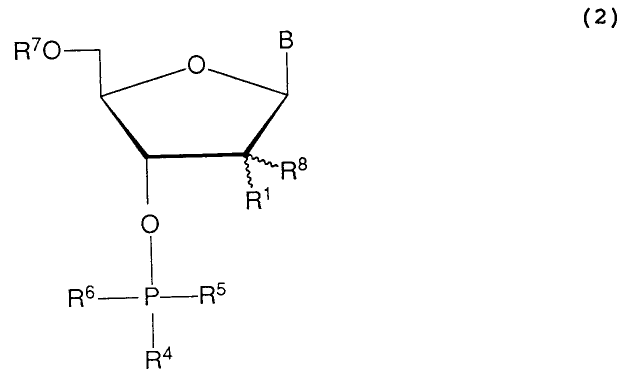 wherein
R1 represents hydrocarbyl residues, such as alkyl, alkenyl and aralkyl, which are optionally substituted at least once by groups possessing functions;
B represents a mono or polynuclear heterocyclic nitrogenous base;
R4, R5 and R6 represent, independently from each other, heteroatom optionally bonded to hydrogen, hydrogen, or residues R1, optionally bonded to phosphor by heteroatom, one of these residues being optionally absent;
R7 represents hydrogen or a protective group; and
R8 represents heteroatom optionally bonded to hydrogen, hydrogen or residues R1 optionally bonded to carbon by heteratom,
by the fact that they have a 2'R or 2'S configuration, and by the fact that they can be used as starting products in the synthesis of the 2'-deoxy-2'-hydrocarbyl-oligonucleotides of claims 1 to 10.

12. Compounds according to claim 11, in which the residue R1 is preferably chosen from the group comprising methyl, ethyl, propyl, allyl and benzyl.

13. Compounds according to claims 11 or 12, in which the residue R1 is substituted with functional groups chosen from among -NH₂, -OH, and -CO.

14. Compounds according to any one of claims 11 to 13, in which B represents a purine derivative.

15. Compounds according to claim 14, in which the purine derivative is chosen from the group comprising adenine, hypoxanthine and guanine.

16. Compounds according to any one of claims from 11 to 13, in which B represents a pyrimidine derivative.

17. Compounds according to claim 16, in which the pyrimidine derivative is chosen from the group comprising cytosine, uracil and thymine.

18. Compounds according to any one of claims 11 to 17, in which the heteroatom represented by R4, R5 and R6, independently from each other, is chosen from the group comprising oxygen and sulphur.

19. Compounds according to any one of claims 11 to 18, in which among the heteroatoms which bond the residues of type R1 to the phosphorus, the oxygen atom is preferred to represent the residues R4, R5 and R6.

20. Compounds according to any one of the claims 11 to 19, in which the groups R1 and B are protected.

21. Compounds according to claim 20, in which the protective group is benzoyl.

22. Compounds according to any one of claims 11 to 20, in which the protective group represented by R7 is 4,4'-dimethoxytriphenylmethyl.

23. Process for the synthesis of the 2'-deoxy-2'-C-hydrocarbyl-oligonucleotides according to claims 1 to 10, starting from the compounds according to claims 11 to 22.
